(19) Europäisches Patentamt
European Patent Office
Office européen des brevets

(11) Publication number: **0 034 430**
**B1**

(12) **EUROPEAN PATENT SPECIFICATION**

(45) Date of publication of patent specification: **05.10.83**

(21) Application number: **81300425.6**

(22) Date of filing: **02.02.81**

(51) Int. Cl.³: **C 07 C 45/49**, C 07 C 47/228, B 01 J 31/28

(54) **Process for preparing arylacetaldehydes.**

(30) Priority: **08.02.80 JP 15173/80**

(43) Date of publication of application: **26.08.81 Bulletin 81/34**

(45) Publication of the grant of the patent: **05.10.83 Bulletin 83/40**

(84) Designated Contracting States: **BE CH DE FR GB IT LI NL SE**

(56) References cited:
**DE - B - 1 955 828**
**FR - A - 2 022 880**

**JOURNAL OF ORGANOMETALLIC CHEMISTRY, Vol. 193, No. 3, 1980 Lausanne F. UNGVARY et al. "Stoichiometric Hydrodehalogenation and Formylation of Organic Halides Using Tetracarbonyl Cobaltate (—1)" pages 379 to 382**

**CHEMISCHES ZENTRALBLATT, Vol. 135, No. 1, 1964, Berlin L. MARKO et al. "Herstellung von Aldehyden" page 255, column 1, abstract No. 2405**

(73) Proprietor: **SUMITOMO CHEMICAL COMPANY, LIMITED**
**15 Kitahama 5-chome Higashi-ku**
**Osaka-shi Osaka-fu (JP)**

(72) Inventor: **Takano, Tetsuo**
**1-26-103, Tamagawa**
**Takatsuki Osaka (JP)**
Inventor: **Suzukamo, Gohu**
**2-1, Kuwata-cho**
**Ibaraki Osaka (JP)**
Inventor: **Ishino, Masaru**
**1-26-309, Tamagawa**
**Takatsuki Osaka (JP)**
Inventor: **Ikimi, Kiyoshi**
**2-13, Kagamida Oyamazaki-cho**
**Otokuni-gun Kyoto (JP)**

(74) Representative: **Allard, Susan Joyce et al, BOULT, WADE & TENNANT 27 Furnival Street London EC4A 1PQ (GB)**

**EP 0 034 430 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Process for preparing arylacetaldehydes

The present invention relates to a process for preparing arylacetaldehydes from arylmethyl halides by their reaction with carbon monoxide and hydrogen.

Arylacetaldehydes are useful as perfumes and also as intermediates in the production of agricultural chemicals and pharmaceuticals.

For the production of arylacetaldehydes by the reaction of arylmethyl halides with carbon monoxide and hydrogen in the presence of a catalyst, a process is disclosed in *Hung. P. 150,412 (Chemical Abstracts, 60, 2847e, (1964).* This process, however, requires a large quantity of catalyst, and still the yield of the desired compound is poor. Also, Japanese Patent Publication (unexamined) No. 144,503/1978 discloses the production of phenylacetaldehyde by reacting benzyl chloride with carbon monoxide and hydrogen in the presence of octacarbonyl dicobalt and an N,N-disubstituted acid amide. However, the yield of the product in this process is at the most about 50% even when a large amount of the catalyst is used.

We have now found that the reaction of aryl-methyl halides with carbon monoxide and hydrogen in the presence of a catalyst together with an inorganic base as appropriately selected provides arylacetaldehydes in high yields with the use of only a small amount of the catalyst.

According to this invention, there is provided a process for preparing an arylacetaldehyde by reation of an arylmethyl halide which is a monocyclic or polycyclic, condensed or non-condensed aromatic compound having at least one halomethyl group on the aromatic ring which may be substituted with one or more substituents selected from $C_1$—$C_6$ alkyl, $C_1$—$C_6$ alkoxy and halogen, with carbon monoxide and hydrogen in the presence of a cobalt compound and a basic reagent in a liquid medium, characterized in that the basic reagent is an inorganic alkali metal or alkaline earth metal compound and the liquid medium is a solvent system which is a nitrile, or a mixture thereof with a hydrocarbon.

Preferred arylmethyl halides may be represented by the formula:

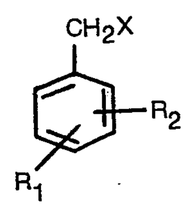

wherein $R_1$ and $R_2$, which may be same or different, are each hydrogen, $C_1$—$C_6$ alkyl, $C_1$—$C_6$ alkoxy or halogen (particularly chlorine) and X is halogen (particularly chlorine).

Specific examples of the arylmethyl halide (I) are benzyl chloride p-fluorobenzyl chloride, p-chlorobenzyl chloride, p-methylbenzyl chloride, p-ethylbenzyl chloride, p-isopropylbenzyl chloride, p-tert-butylbenzyl chloride, p-methoxybenzyl chloride, p-ethoxybenzyl chloride, and o,p-di-methylbenzyl chloride.

The cobalt compound used as the catalyst may be any that is conventionally employed for carbonylation and hydroformylation, which includes complexes having carbon monoxide, phosphines or amines as ligands, carboxylates, hydroxides, halides, oxides and nitrates. Specific examples are cobalt carbonyls, cobalt hydroxide, cobalt carbonate, cobalt acetate, cobalt formate, cobalt naphthenate and cobalt bromide. Particularly preferred are cobalt carbonyls, which include octacarbonyl dicobalt, dodecacarbonyl tetracobalt, hydridotetracarbonyl cobalt and hexacarbonyl bis(tri-n-butylphosphine)dicobalt. In the reaction system in which the basic reagent coexists, these cobalt carbonyls may substantially be converted to cobalt carbonyl anions, for example, the alkali metal salt of tetracarbonyl cobalt which may be catalytically active. A preferred range of the amount of the catalyst is from $10^{-4}$ to $10^{-1}$ gram-atom (calculated as metallic cobalt) based on one mole of the arylmethyl halide.

The inorganic alkali metal or alkaline earth metal compound usable as the basic reagent may be an inorganic compound of an alkali metal which is alkaline at room temperature (e.g. about 5 to 30°C) in aqueous solution. Specific examples are oxides, hydroxides, carbonates, phosphates, silicates and borates. Preferred are the carbonates of alkali metals, and particularly preferred are sodium carbonate and potassium carbonate. The amount of the inorganic alkali metal compound may be equivalent to or slightly in excess of one mole of the arylmethyl halide.

The solvent system, which is preferably used in the process of the invention is a nitrile or a mixture thereof with hydrocarbon. Examples of nitriles are acetonitrile, benzonitrile, etc. Examples of hydrocarbons are those having not more than 12 carbon atoms such as cyclohexane. The concentration of the arylmethyl halide in these solvent systems is usually from 1 to 80% by weight, preferably from 5 to 50% by weight.

Carbon monoxide and hydrogen may be introduced into the reaction system separately but they are usually introduced as a mixture. The molar ratio of carbon monoxide to hydrogen in the mixture is normally 1 or more, preferably from 1 to 5, so that the desired compound will be produced in a high selectivity. The mixture is normally used at a pressure of not less than 10 atm (10.13 bar), preferably from 50 to 300 atm (50.65 to 303.9 bar). The mixture need not be pure; for example, it may contain an inert gas such as nitrogen or helium.

The reaction temperature is not particularly limited, but a temperature in the range of from 50 to 200°C, preferably of from 80 to 160°C, may be employed. The reaction time depends upon the other reaction conditions such as the retention temperature, but a period of from 0.5 to 10 hours is generally adopted.

The reaction may be carried out batchwise or continuously. In order to prevent the reaction system from contamination with metal compounds which may dissolve out of a reactor wall the use of a reactor of which the inside wall is protected by a glass-lining or an anticorrosive material, such as Hastelloy, is desired.

The thus produced arylacetaldehyde may be separated from the reaction mixture and purified by per se conventional techniques such as distillation and extraction.

The arylacetaldehyde produced corresponds substantially to the starting arylmethyl halide. The arylacetaldehyde is an aromatic compound having at least one formylmethyl group on the aromatic ring. The aromatic ring may be monocyclic or polycyclic and also condensed or non-condensed. There may also be present on the aromatic ring, in addition to the formylmethyl group(s), any other substituent(s) which are same as or different from those present in the starting arylmethyl halide. Examples of such substituent(s) are $C_1$—$C_6$ alkyl, $C_1$—$C_6$ alkoxy, halogen (e.g. chlorine, bromine, fluorine, iodine), etc. Preferred arylacetaldehydes may be represented by the formula:

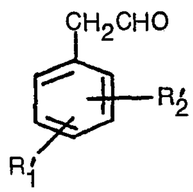

(II)

wherein $R_1'$ and $R_2'$, which may be same or different, are each hydrogen, $C_1$—$C_6$ alkyl, $C_1$—$C_6$ alkoxy or halogen (particularly chlorine).

Specific examples of the arylacetaldehyde (II) are phenylacetaldehyde, p-fluorophenylacetaldehyde, p-chlorophenylacetaldehyde, p-methylphenylacetaldehyde, p-ethylphenylacetaldehyde, p-isopropylphenylacetaldehyde, p-tertbutylphenylacetaldehyde, p-methoxyphenylacetaldehyde, p-ethoxyphenylacetaldehyde, o,p-dimethylphenylacetaldehyde, etc.

The process of this invention will be illustrated specifically with reference to the following examples, which are not however to be interpreted as limiting the invention thereto.

Identification and determination of the product were made by IR spectrum, NMR spectrum and gas chromatography. The conversion, selectivity and yield of the product are based on the following equations:

$$\text{Conversion} = \frac{\text{Moles of arylmethyl halide consumed}}{\text{Moles of arylmethyl halide used}} \times 100\ (\%)$$

$$\text{Selectivity} = \frac{\text{Moles of arylacetaldehyde produced}}{\text{Moles of arylmethyl halide consumed}} \times 100\ (\%)$$

$$\text{Yield} = \frac{\text{Moles of arylacetaldehyde produced}}{\text{Moles of arylmethyl halide used}} \times 100 (\%)$$

Example 1

Into a 50 ml autoclave, there were charged p-methylbenzyl chloride (0.70 g), octacarbonyl dicobalt (0.068 g), sodium carbonate (0.60 g) and acetonitrile (10 ml). After replacing air in the autoclave by nitrogen, a mixed gas comprising carbon monoxide and hydrogen in a molar ratio of 1:4 was introduced therein to make a pressure of 100 atm (101.3 bar). The contents of the autoclave were heated at 100°C for 5 hours with stirring, cooled to room temperature and analyzed by gas chromatography to obtain the following results: conversion of p-methylbenzyl chloride, 100%; yield of p-methylphenylacetaldehyde, 91.0%.

### Reference Example 1

In the same manner as in Example 1 but using methylethylketone (10 ml) in place of acetonitrile, the reaction was carried out. The results were as follows: conversion of p-methylbenzyl chloride, 73.6%; yield of p-methylphenylacetaldehyde, 43.7%.

### Example 2

Into the same autoclave as in Example 1, there were charged p-methylbenzyl chloride (1.40 g), octacarbonyl dicobalt (0.034 g), sodium carbonate (1.10 g), cyclohexane (10 ml) and acetonitrile (1 ml). After replacing air in the autoclave by nitrogen, a mixed gas comprising carbon monoxide and hydrogen in a molar ratio of 1:4 was introduced therein to make a pressure of 100 atm (101.3 bar). The contents of the autoclave were heated at 100°C for 3 hours with stirring, cooled to room temperature and analyzed by gas chromatography to obtain the following results: conversion of p-methylbenzyl chloride, 99.7%; yield of p-methylphenylacetaldehyde, 90.1%. p-Xylene was by-produced in an amount of 8.8 mole % based on p-methylbenzyl chloride used.

### Examples 3 to 4 and Reference Example 2

In the same manner as in Example 1 but using benzyl chloride (0.63 g) and various kinds of inorganic bases, the reaction was carried out. The results were as shown in Table 1.

TABLE 1

| Example No. | Inorganic base | Conversion of benzyl chloride (%) | Phenylacetaldehyde | |
|---|---|---|---|---|
| | | | Yield (%) | Selectivity (%) |
| 3 | Sodium carbonate (0.60 g) | 86.6 | 76.5 | 88.3 |
| 4 | Potassium carbonate (0.70 g) | 81.5 | 70.5 | 86.5 |
| Reference 2 | Sodium acetate (0.82 g) | 17.8 | 12.4 | 69.6 |

Example 5

In the same manner as in Example 1 but using benzyl bromide (0.90 g) and adopting a reaction time of 2 hours, the reaction was carried out. The results were as follows: conversion of benzyl bromide, 92.7%; selectivity to phenylacetaldehyde, 73.7%; yield of phenylacetaldehyde 68.3%.

Example 6

In the same manner as in Example 1 but using o-chlorobenzyl chloride (0.81 g), the reaction was carried out. The results were as follows: conversion of o-chloro-benzyl chloride, 90.6%; selectivity to o-chlorophenyl-acetaldehyde, 74.5%; yield of o-chlorophenylacetaldehyde, 67.5%.

**Claims**

1. A process for preparing an arylacetaldehyde by reacting an arylmethyl halide which is a monocyclic or polycyclic, condensed or non-condensed aromatic compound having at least one halomethyl group on the aromatic ring which may be substituted with one or more substituents selected from $C_1$—$C_6$ alkyl, $C_1$—$C_6$ alkoxy and halogen, with carbon monoxide and hydrogen in the presence of a cobalt compound and a basic reagent in a liquid medium, characterised in that the basic reagent is an alkali metal or alkaline earth metal compound and the liquid is a solvent system which is a nitrile or a mixture thereof with a hydrocarbon.

2. A process as claimed in claim 1 wherein the arylmethyl halide is a compound of the formula:

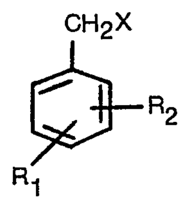

wherein $R_1$ and $R_2$, which may be same or different, are each hydrogen, $C_1$—$C_6$ alkyl, $C_1$—$C_6$ alkoxy or halogen and X is halogen.

3. A process as claimed in claim 2 wherein the arylmethyl halide is benzyl chloride, p-fluorobenzyl chloride, p-chlorobenzyl chloride, p-methylbenzyl chloride, p-ethyl-benzyl chloride, p-isopropylbenzyl chloride, p-tert-butyl-benzyl chloride, p-methoxybenzyl chloride, p-ethoxybenzyl chloride or o,p-dimethylbenzyl chloride.

4. A process as claimed in any one of the preceding claims wherein the cobalt compound is a carboxylate, hydroxide, halogenide, oxide or nitrate or a complex in which carbon monoxide, phosphine or an amine is a ligand.

5. A process as claimed in claim 4 wherein the cobalt compound is octacarbonyl dicobalt, dodecarcarbonyl tetracobalt, hydridotetracarbonyl cobalt or hexacarbonyl bis (tri-n-butylphosphine)dicobalt.

6. A process as claimed in any one of the preceding claims wherein the amount of the cobalt compound added is from $10^{-4}$ to $10^{-1}$ gram-atom (calculated as metallic cobalt) based on one mole of the arylmethyl halide.

7. A process as claimed in any one of the preceding claims wherein the inorganic alkali metal or alkaline earth metal compound is an oxide, hydroxide, carbonate, phosphate, silicate or borate.

8. A process as claimed in claim 7 wherein the inorganic alkali metal compound is the carbonate of an alkali metal.

9. A process as claimed in claim 8 wherein the carbonate of the alkali metal is sodium carbonate or potassium carbonate.

10. A process as claimed in any one of the preceding claims wherein the amount of the inorganic alkali metal compound is equivalent to or slightly in excess of one mole of the arylmethyl halide.

11. A process as claimed in any one of the preceding claims wherein the hydrocarbon solvent has not more than 12 carbon atoms.

12. A process as claimed in any one of the preceding claims wherein the concentration of the arylmethyl halide in the solvent system is from 1 to 80% by weight.

13. A process as claimed in any one of the preceding claims wherein the molar ratio of carbon monoxide to hydrogen is 1 or more.

14. A process as claimed in any one of the preceding claims wherein the reaction is carried out under a pressure of 10 atms 10.13 bar or more.

15. A process as claimed in any one of the preceding claims wherein the reaction is carried out at a temperature in the range of from 50 to 200°C.

16. A process as claimed in any one of the preceding claims wherein the reaction is carried out for a period of from 0.5 to 10 hours.

**Revendications**

1. Procédé pour préparer un arylacétaldéhyde en faisant réagir un halogénure d'arylméthyle qui est un composé aromatique monocyclique ou polycyclique, condensé ou non condensé, ayant au moins un groupe halométhyle sur le noyau aromatique, qui peut être substitué par un ou plusieurs substituants choisis parmi des groupes alkyles en $C_1$—$C_6$, alcoxy en $C_1$—$C_6$ et des halogènes, avec de l'oxyde de carbone et de l'hydrogène, en présence d'un composé de cobalt et d'un réactif basique dans un milieu liquide, caractérisé en ce que le réactif basique est un composé de métal alcalin ou de mètal alcalino-terreux et le liquide est un système de solvants qui est un nitrile ou son mélange avec un hydrocarbure.

2. Procédé selon la revendication 1, dans lequel l'halogénure d'arylméthyle est un composé de formule:

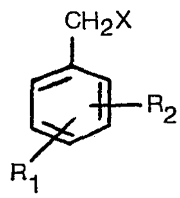

où $R_1$ et $R_2$, qui peuvent être les mêmes ou différents, représentent chacun l'hydrogène, un groupe alkyle en $C_1$—$C_6$, alcoxy en $C_1$—$C_6$ ou un halogène et X est un halogène.

3. Procédé selon la revendication 2, dans lequel l'halogénure d'arylméthyle est le chlorure de benzyle, le chlorure de p-fluorobenzyle, le chlorure de p-chlorobenzyle, le chlorure de p-méthylbenzyle, le chlorure de p-éthylbenzyle, le chlorure de p-isopropylbenzyle, le chlorure de p-t-butylbenzyle, le chlorure de p-méthoxybenzyle, le chlorure de p-éthoxybenzyle ou le chlorure d'o,p-diméthylbenzyle.

4. Procédé selon l'une quelconque des revendications précédentes, dans lequel le composé de cobalt est un carboxylate, un hydroxyde, un halogénure, un oxyde ou un nitrate ou un complexe dans lequel l'oxyde de carbone, la phosphine ou une amine est un coordinat.

5. Procédé selon la revendication 4, dans lequel le composé de cobalt est l'octacarbonyldicobalt, le dodécacarbonyltétracobalt, l'hydrurotétracarbonylcobalt ou l'hexacarbonylbis(tri-n-butylphosphine)-dicobalt.

6. Procédé selon l'une quelconque des revendications précédentes, dans lequel la quantité du composé de cobalt ajoutée est de $10^{-4}$ à $10^{-1}$ atome gramme (calculée sous forme de cobalt métallique) en se basant sur une mole de l'halogénure d'arylméthyle.

7. Procédé selon l'une quelconque des revendications précédentes, dans lequel le composé minéral de métal alcalin ou de métal alcalino-terreux est un oxyde, un hydroxyde, un carbonate, un phosphate, un silicate ou un borate.

8. Procédé selon la revendication 7, dans lequel le composé minéral du métal alcalin est le carbonate d'un métal alcalin.

9. Procédé selon la revendication 8, dans lequel le carbonate du métal alcalin est le carbonate de sodium ou le carbonate de potassium.

10. Procédé selon l'une quelconque des revendications précédentes, dans lequel la quantité du composé minéral de métal alcalin est équivalente à ou légèrement en excès par rapport à une mole de l'halogénure d'arylméthyle.

11. Procédé selon l'une quelconque des revendications précédentes, dans lequel le solvant hydrocarboné n'a pas plus de 12 atomes de carbone.

12. Procédé selon l'une quelconque des revendications précédentes, dans lequel la concentration de l'halogénure d'arylméthyle dans le système de solvants est 1 à 80% en poids.

13. Procédé selon l'une quelconque des revendications précédentes, dans lequel le rapport molaire oxyde de carbone/hydrogène est 1 ou plus.

14. Procédé selon l'une quelconque des revendications précédentes, dans lequel la réaction est réalisée sous une pression de 10 atmosphères (10, 13 bars) ou plus.

15. Procédé selon l'une quelconque des revendications précédentes, dans lequel la réaction est réalisée à une température dans l'intervalle de 50 à 200°C.

16. Procédé selon l'une quelconque des revendications précédentes, dans lequel la réaction est réalisée pendant une période de 0,5 à 10 heures.

**Patentansprüche**

1. Verfahren zur Herstellung eines Arylacetaldehyds durch Umsetzung eines Arylmethylhalogenids, das eine mono- oder polycyclische, kondensierte oder nichtkondensierte aromatische Verbindung darstellt, die mindestens eine Halogenmethylgruppe am aromatischen Ring aufweist, der

gegebenenfalls mit einem oder mehreren Substituenten aus der Gruppe $C_1$—$C_6$-Alkylrest, $C_1$—$C_6$-Alkoxyrest und Halogenatom substituiert sein kann, mit Kohlenmonoxid und Wasserstoff in Gegenwart einer Kobaltverbindung und eines basischen Reagens in flüssigem Medium, dadurch gekennzeichnet, daß man als basisches Reagens eine Alkalimetall- oder Erdalkalimetallverbindung einsetzt und als Flüssigkeit ein Lösungsmittelsystem einsetzt, das aus einem Nitril oder dessen Gemisch mit einem Kohlenwasserstoff besteht.

2. Verfahren nach Anspruch 1, dadurch gekennzeichnet, daß man als Arylmethylhalogenid eine Verbindung der Formel

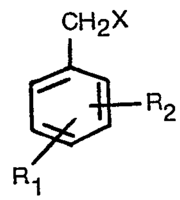

einsetzt, in der $R_1$ und $R_2$ gleich oder verschieden sind und jeweils ein Wasserstoffatom, $C_1$—$C_6$-Alkylrest, $C_1$—$C_6$-Alkoxyrest oder ein Halogenatom bedeuten und X ein Halogenatom darstellt.

3. Verfahren nach Anspruch 2, dadurch gekennzeichnet, daß man als Arylmethylhalogenid Benzylchlorid, p-Fluorbenzylchlorid, p-Chlorbenzylchlorid, p-Methylbenzylchlorid, p-Äthylbenzylchlorid, p-Isopropylbenzylchlorid, p-tert.-Butylbenzylchlorid, p-Methoxybenzylchlorid, p-Äthoxybenzylchlorid oder o,p-Dimethylbenzylchlorid einsetzt.

4. Verfahren nach jedem der vorstehenden Patentansprüche, dadurch gekennzeichnet, daß man als Kobaltverbindung ein Carboxylat, Hydroxid, Halogenid, Oxid oder Nitrat oder einen Komplex, in dem Kohlenmonoxid, Phosphin oder ein Amin einen Liganden darstellt, einsetzt.

5. Verfahren nach Anspruch 4, dadurch gekennzeichnet, daß man als Kobaltverbindung Octacarbonyldikobalt, Dodecacarbonyltetrakobalt, Hydridotetracarbonylkobalt oder Hexacarbonylbis-(tri-n-butylphosphin)-dikobalt einsetzt.

6. Verfahren nach jedem der vorstehenden Patentansprüche, dadurch gekennzeichnet, daß man $10^{-4}$ bis $10^{-1}$ Grammatom Kobaltverbindung (berechnet als metallisches Kobalt), bezogen auf 1 Mol des Arylmethylhalogenids, zugibt.

7. Verfahren nach jedem der vorstehenden Patentansprüche, dadurch gekennzeichnet, daß man als anorganische Alkalimetall- oder Erdalkalimetallverbindung ein Oxid, Hydroxid, Carbonat, Phosphat, Silikat oder Borat einsetzt.

8. Verfahren nach Anspruch 7, dadurch gekennzeichnet, daß man als anorganische Alkalimetallverbindung ein Alkalimetallcarbonat einsetzt.

9. Verfahren nach Anspruch 8, dadurch gekennzeichnet, daß man als Alkalimetallcarbonat Natriumcarbonat oder Kaliumcarbonat einsetzt.

10. Verfahren nach jedem der vorstehenden Patentansprüche, dadurch gekennzeichnet, daß die Zugabemenge der anorganischen Alkalimetallverbindung der Menge von 1 Mole des Arylmethylhalogenids entspricht oder in einem geringfügigen Überschuß gegenüber dem Arylmethylhalogenid vorliegt.

11. Verfahren nach jedem der vorstehenden Patentansprüche, dadurch gekennzeichnet, daß das Kohlenwasserstoff-Lösungsmittel nicht mehr als 12 Kohlenstoffatome enthält.

12. Verfahren nach jedem der vorstehenden Patentansprüche, dadurch gekennzeichnet, daß die Konzentration des Arylmethylhalogenids im Lösungsmittelsystem 1 bis 80 Gewichtsprozent beträgt.

13. Verfahren nach jedem der vorstehenden Patentansprüche, dadurch gekennzeichnet, daß das Molverhältnis von Kohlenmonoxid zu Wasserstoff 1 oder mehr ist.

14. Verfahren nach jedem der vorstehenden Patentansprüche, dadurch gekennzeichnet, daß man die Umsetzung bei Drucken von 10 at (10, 13 bar) oder mehr durchführt.

15. Verfahren nach jedem der vorstehenden Patentansprüche, dadurch gekennzeichnet, daß man die Umsetzung bei Temperaturen in einem Bereich von 50 bis 200°C durchführt.

16. Verfahren nach jedem der vorstehenden Patentansprüche, dadurch gekennzeichnet, daß man die Umsetzung während einer Zeitdauer von 0,5 bis 10 Stunden durchführt.